# EUROPEAN PATENT APPLICATION

(11) **EP 2 386 541 A1**
(43) Date of publication of application: **16.11.2011**
(21) Application number: 11156932.3
(22) Date of filing: 04.03.2011
(51) Int. Cl.: C07D 209/14, C07D 209/40

(54) **Novel methods for the preparation of P2X7R antagonists**

(30) Priority: 14.05.2010 EP 10162812
(71) Applicant: Affectis Pharmaceuticals AG, 82152 Martinsried (DE)
(72) Inventor: Bös, Michael, 80796 Munich (DE)
(74) Representative: Benz, Jürgen

(57) **Abstract**

Disclosed are novel methods for the synthesis of N-substituted indol-3-yl-alkylamide compounds which act as P2X7R antagonists, said methods involving the rearrangement of an oxime intermediate.

## Description

The present application relates to novel methods for the synthesis of N-substituted indol-3-yl-alkylamide compounds which act as P2X7R antagonists.

### BACKGROUND

P2X7R is an ATP-gated ion channel belonging to the P2X ionotropic channel family. The gene was first isolated from rat brain (Surprenant et al. (1996) 272:735-738) and subsequently from a human monocyte library (Rassendren et al. (1997) J. Biol. Chem. 272:5482-5486; Genbank accession numbers NM_002562, Y09561) by virtue of its sequence homology with the other members of the P2X family. It was later found that P2X7R corresponded to the unidentified P2Z receptor which mediates the permeabilising action of ATP on mast cells and macrophages (Dahlqvist and Diamant (1974) Acta Physiol. Scand. 34:368-384; Steinberg and Silverstein (1987) J. Biol. Chem. 262:3118-3122; Gordon (1986) Biochem. J. 233:309-319). The P2X7R has two hydrophobic membrane-spanning domains, an extracellular loop, and forms transmembrane ion channels. P2X7R bears a pharmacological profile markedly different from other P2X homo- or heteromers (North and Surprenant (2000) Annual Rev. Pharmacology Toxicology 40:563-580). P2X7R requires levels of ATP in excess of 1 mM to achieve activation, whereas other P2X receptors activate at ATP concentrations of ≤100 *µ*m (Steinberg et al. (1987) J. Biol. Chem. 262:8884-8888; Greenberg et al. (1988) J. Biol. Chem. 263:10337-10343). While all P2X receptors demonstrate non-selective channel-like properties following ligation, the channels formed by the P2X7R can rapidly transform into pores that can allow the passage of molecules of up to 900 Dalton (Virginio et al. (1999) J. Physiol. 519:335-346).

P2X7R is expressed in haematopoietic cells, mast cells, lymphocytes, erythrocytes, fibroblast, Langerhans cells, and macrophages (Surprenant et al., 1996, Science 272:3118-3122). In the central nervous system, P2X7R expression has been reported in glial cells, Schwann cells, astrocytes, as well as in neurons (Ferrari et al. (1996) J. Immunol 156:1531-1539; Collo et al. (1997) Neuropharmacology 36: 1277-1283; Anderson and Nedergaard (2006) Trends Neuroscien 29: 257-262).

P2X7R is involved in the regulation of the immune function and inflammatory response. Activation of P2X7R by ATP in macrophages is associated with mitogenic stimulation of T cells (Baricordi et al. (1996) Blood 87:682-690), the release of cytokines (Griffiths et al. (1995) J. Immol. 154:2821-2828), and formation of macrophage polykarions (Falzoni et al. (1995) J. Clin. Invest. 95:1207-1216). P2X7R is involved in the processing and release of active interleukin-1 beta (IL-1ß) from proinflammatory cells (Perregaux and Gabel (1998) J Biol Chem 269:15195-15203; Ferrari et al., (2006) J Immunol 176: 3877-3883). Stimulation of the P2X7R by ATP can also result in apoptosis and cell death by triggering the formation of non-selective plasma membrane pores (Di Virgilio et al. (1998) Cell Death Differ. 5:191-199).

Upregulation of P2X7R has been observed during ischemic damage and necrosis induced by occlusion of middle cerebral artery in rat brain (Collo et ai. (1997) Neuropharmacol 36:1277-1283). Recent studies indicate a role of P2X7R in the generation of superoxide in microglia, and upregulation of P2X7R has been detected around amyioid plaques in a transgenic mouse models for Alzheimer's disease (Parvathenani et al. (2003) J Biol Chem 278:13300-13317) and in multiple sclerosis lesions from autopsy brain sections (Narcisse et al. (2005) Glia, 49:245-258).

Studies from mice lacking P2X7R resulted in absence of inflammatory and neuropathic hypersensitivity to mechanical and thermal stimuli, indicating a link between P2X7R and inflammatory and neuropathic pain (Chessell et al. (2005) Pain 114:386-396). Antagonists of P2X7R significantly improved functional recovery and decreased cell death in spinal cord injury in animal models (Wang et al. (2004) Nature Med 10:B21-B27).

Compounds which modulate P2X7R have been reported. For example, Brilliant Blue (Jiang et al., Mol. Phamacol. 58 (2000), 82-88), the isoquinolines 1-[N,O-Bis(5-isoquinolinesulfonyl)-N-methyl-L-tyrosyl]-4- phenylpiperazine and N-[1-[N-methyl-p-(5 isoquinolinesulfonyl) benzyl]-2-(4-phenylpiperazine)ethyl]-5-isoquinolinesulfonamide(Humphreys et al., Mol. Pharmacol., 54 (1998), 22-32), adamantane derivatives (WO 99/29060, WO 99/29661, WO 00/61569, WO 01/42194, WO 01/44170, WO 01/44213, WO 01/94338, WO 03/041707, WO 03/042190, WO 03/080579, WO 04/074224, WO 05/014529, WO 06/025783, WO 06/059945), pipendine and piperazine compounds (WO 01/44213, WO 01/46200, WO 08/005368), benzamide and heteroarylamide compounds (WO 03/042191, WO 04/058731, WO 04/058270, WO 04/099146, WO 05/019182, WO 06/003500, WO 06/003513, WO 06/067444), substituted tyrosine derivatives (WO 00/71529, WO 03/047515, WO 03/059353), imidazole compounds (WO 05/014555), amino-tetrazoles compounds (WO 05/111003), cyanoamidine (WO 06/017406), bicycloheteroaryl derivatives (WO 05/009968, WO 06/102588, WO 06/102610, WO 07/028022, WO 07/109154, WO 07/109160, WO 07/109172, WO 07/109182, WO 07/109192, WO 07/109201), acylhydrazide (WO 06/110516), and other examples (WO 99/29686, WO 04/106305, WO 05/039590, WO 06/080884, WO 06/086229, WO 06/136004, WO 07/025366, WO 07/056046, WO 07/056091, WO 07/141267, WO 07/141269, WO 08/003697) are antagonists of P2X7R while Oxidized ATP (oATP) acts as an irreversible inhibitor of the receptor (Chen et al., J. Biol. Chem., 268 (1993), 8199-8203).

Consequently, there is strong evidence that compounds acting on P2X7R can be used in the treatment of pain, inflammatory processes, and degenerative conditions associated with disease states such as rheumatoid arthritis, osteoarthritis, psoriasis, allergic dermatitis, asthma, chronic obstructive pulmonary disease, airways hyper-responsiveness, septic shock, glomerulonephritis, irritable bowel disease, inflammatory bowel disease, Crohn's disease, ulcerative colitis, atherosclerosis, growth and metastases of malignant cells, myoblastic leukaemia, diabetes, Alzheimer's disease, Parkinson's disease, multiple sclerosis, glaucoma, age-related macular degeneration, uveitis, neuropathic pain, depression, bipolar affective disorders, anxiety, meningitis, traumatic brain injury, acute spinal cord injury, neuropathic pain, osteoporosis, burn injury, ischemic heart disease, myocardial infarction, stroke, and varicose veins.

A series of compounds which inhibit P2X7R activity and can thus be used in the treatment of the above mentioned diseases has been reported in EP application No. 10156190. The present invention relates to a novel method which allows compounds of this series to be prepared conveniently at high yields.

### SUMMARY OF THE INVENTION

In a first aspect, the present invention provides a process for the preparation of a compound of formula (I) 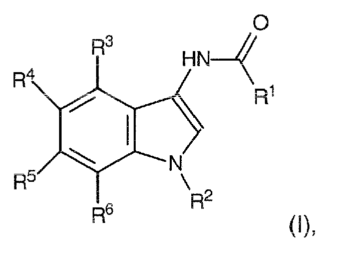 wherein R¹ is a hydrocarbyl group, R³, R⁴, R⁵ and R⁶ are independently selected from H, alkyl, alkoxy, CN or CF₃ and halogen, and R² is an alkyl group which is optionally substituted by hydroxy, alkoxy, halogen, -NR⁷R⁸, CN or CF₃, with R⁷ and R⁸ being independently selected from H or alkyl, or of a pharmaceutically acceptable salt thereof,
comprising the rearrangement reaction of a compound of formula (II) 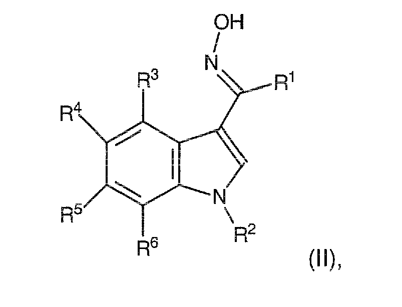 wherein R¹, R², R³, R⁴, R⁵ and R⁶ are defined as above.

According to a preferred aspect of the invention, the compound of formula (II) is prepared by a process comprising a step of reacting a compound of formula (III) 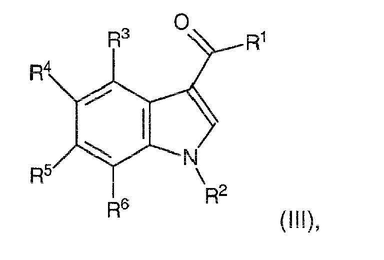 with hydroxylamine,
wherein, in formula (III), R¹ is a hydrocarbyl group, R³, R⁴, R⁵ and R⁶ are independently selected from H, alkyl, alkoxy, CN or CF₃ and halogen, and R² is an alkyl group which is optionally substituted by hydroxy, alkoxy, halogen, -NR⁷R⁸, CN or CF₃, with R⁷ and R⁸ being independently selected from H or alkyl.

In a further preferred aspect, the compound of formula (III) is prepared by a process comprising a step of subjecting a compound of formula (IV) 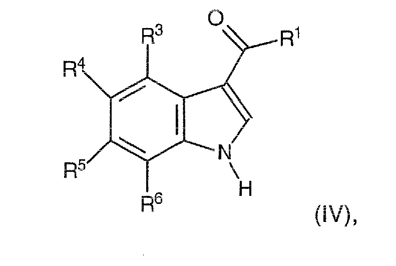 wherein R¹ and R³ to R⁶ are defined as above, to a reaction which replaces the hydrogen substituent on the ring N-atom with a group R², to yield a compound of formula (III), 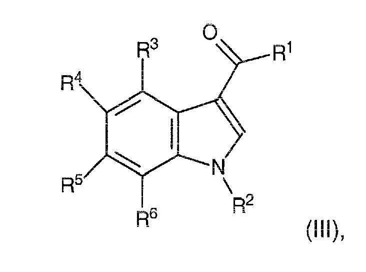 wherein R¹ is a hydrocarbyl group, R³, R⁴, R⁵ and R⁶ are independently selected from H, alkyl, alkoxy, CN or CF₃ and halogen, and R² is an alkyl group which is optionally substituted by hydroxy, alkoxy, halogen, -NR⁷R⁸, CN or CF₃, with R⁷ and R⁸ being independently selected from H or alkyl.

The process of the invention in accordance with any of the above aspects preferably comprises the acylation of a compound of formula (V) 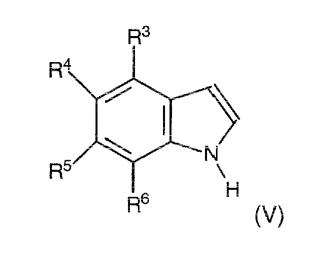 wherein R³, R⁴, R⁵ and R⁶ are independently selected from H, alkyl, alkoxy, CN or CF₃ and halogen, with an organic acid of the formula R¹-C(O)-OH or an activated derivative thereof which contains the acyl group -C(O)-R¹, wherein R¹ is a hydrocarbyl group.

Finally, the invention encompasses compounds of formula (II) as defined above, 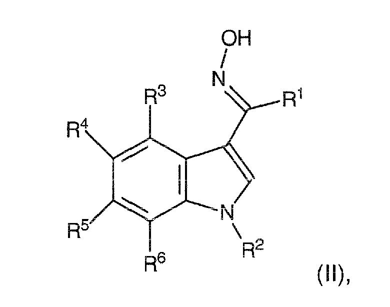 wherein R¹ is a hydrocarbyl group, R³, R⁴, R⁵ and R⁶ are independently selected from H, alkyl, alkoxy, CN or CF₃ and halogen, and R² is an alkyl group which is optionally substituted by hydroxy, alkoxy, halogen, -NR⁷R⁸, CN or CF₃, with R⁷ and R⁸ being independently selected from H or alkyl, as key intermediates of the process in accordance with the invention.

### DETAILED DESCRIPTION OF THE INVENTION

As set out above, the invention provides a process for the preparation of a compound of formula (I) 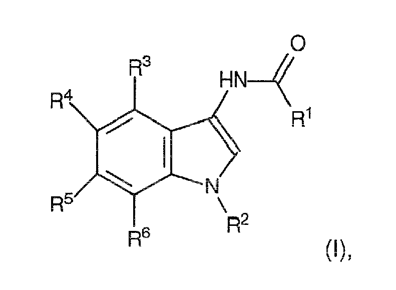 wherein R¹ is a hydrocarbyl group, R³, R⁴, R⁵ and R⁶ are independently selected from H, alkyl, alkoxy, CN or CF₃ and halogen, and R² is an alkyl group which is optionally substituted by hydroxy, alkoxy, halogen, -NR⁷R⁸, CN or CF₃, with R⁷ and R⁸ being independently selected from H or alkyl, or of a pharmaceutically acceptable salt thereof,
comprising the rearrangement reaction of a compound of formula (II) 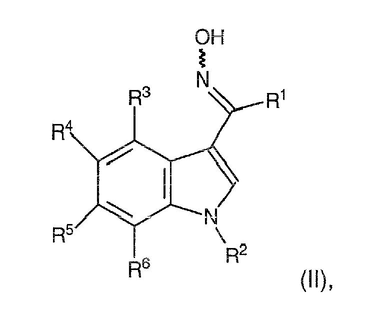 wherein R¹, R², R³, R⁴, R⁵ and R⁶ are defined as above. In accordance with the general practice in the art, the shape of the schematic bond between the N atom and the hydroxyl group of the oxime indicates that the formula embraces the *syn* and the *anti*-form of the oxime as well as mixtures thereof.

It has been found that the yield of the target compounds of formula (I) is optimized by attachment of the substituent R² to the N-atom in the indol ring system before the intermediate compounds of formula (II) are subjected to the rearrangement reaction.

In all compounds referred to herein which contain the group R¹, preferred as groups R¹ are hydrocarbon groups containing up to 20 carbon atoms, in particular up to 10 carbon atoms. In addition, it is preferred that R¹ contains 4 or more, in particular 6 or more carbon atoms. Generally, it is preferred that R¹ is an alkyl group wich may comprise linear, branched or cyclic alkyl moieties. It will be understood that alkyl or cycloalkyl groups containing the above numbers of carbon atoms are further preferred.

It is also preferred for all compounds referred to herein that R¹ has the structure ―CH₂-R^{1a}, wherein R^{1a} is selected from a mono- or bicycloalkyl group, or from a mono- or bicycloalkylalkyl group. The term "mono- or bicycloalkylalkyl" is to be understood in this context as designating a mono- or bicycloalkyl moiety which is bound via an alkylene linker to the remainder of the molecule, i.e. to the ―CH₂- group of the -CH₂-R^{1a} moiety. R^{1a} preferably contains up to 19, in particular up to 9 carbon atoms. As a cyclic alkyl group, it contains 3 or more, preferably 5 or more carbon atoms.

Particularly preferred as R¹ is the group ―CH₂-R^{1a}, wherein R^{1a} is selected from cyclopentyl, cyclopentylmethyl, cyclohexyl, cyclohexylmethyl, cycloheptyl, cycloheptylmethyl, bicyclo[2.2.2]octan-1-yl and bicyclo[2.2.2]octan-1-ylmethyl. As indicated above, reference to a methyl moiety in this context indicates the presence of an additional methylene linker between the ―CH₂- group to which R^{1a} is bound and the respective cycloalkyl moiety. It is further preferred that R^{1a} is selected from cyclopentyl, cyclohexyl, cycloheptyl, and bicyclo[2.2.2]octan-1-yl, and particularly preferred for R^{1a} is cycloheptyl.

In all compounds referred to herein which contain the group R², preferred as R² is an optionally substituted alkyl group having up to 6, in particular up to 4 carbon atoms, such as an opitionally substitiued methyl, ethyl or propyl group. Most preferred is an optionally substituted ethyl group.

Substituted groups R² preferably have one or two, in particular one substituent. An alkoxy substituent of R², if present, preferably contains 1 to 5 carbon atoms. R⁷ and R⁸, if rpresent are preferably H or contain 1 to 5 carbon atoms. Preferred as substituent is a hydroxy group, a halogen or ―NH₂, in particular hydroxy. Thus, particularly preferred as R² is an alkyl group carrying one hydroxy substituent, such as hydroxymethyl, hydroxyethyl or hydroxypropyl. Most preferred is the group ―CH₂-CH₂OH.

In all compounds referred to herein, if any one of R³ to R⁶ is an alkyl or alkoxy group, preferred are groups containing 1 to 5 carbon atoms. Particularly preferred are methyl or methoxy. If anyone of R³ to R⁶ is halogen, preference is given to Cl or Br, particularly Cl.

Generally preferred as R³ to R⁶ are hydrogen, halogen, methyl, methoxy, cyano or trifluoromethyl, and particularly hydrogen, methyl or Cl. Furthermore, preference is given to the case where at least two of R³ to R⁶ are hydrogen, and further preferred is the case where three of R³ to R⁶ are hydrogen, and the remaining one is a non-hydrogen substituent as defined above, in particular halogen, methyl, methoxy, cyano or trifluoromethyl, with a further preference for methyl or Cl. It is particularly preferred that R³ is the substituent which is not hydrogen.

From the above, it will be understood that it is preferred in the context of the invention that the compound of formula (I) has the formula (Ia): 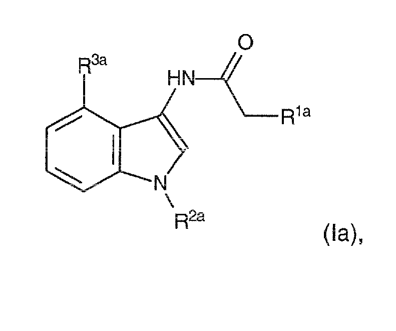 wherein R^{3a} is alkyl, preferably methyl, or halogen, preferably Cl or Br; R^{1a} is selected from cyclopentyl, cyclopentylmethyl, cyclohexyl, cyclohexylmethyl, cycloheptyl, cycloheptylmethyl, bicyclo[2.2.2]octan-1-yl and bicyclo[2.2.2]octan-1-ylmethyl; preferably cyclopentyl, cyclohexyl, cycloheptyl or bicyclo[2.2.2]octan-1-yl; and R^{2a} is an alkyl group which is optionally substituted by hydroxy, preferably ―CH₂OH, -CH₂-CH₂OH or ―CH₂-CH₂-CH₂OH; and is prepared by a process comprising the rearrangement of a compound of formula (IIa): 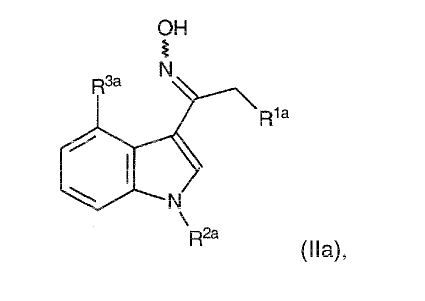 wherein R^{1a}, R^{2a} and R^{3a} are defined as for the compound of formula (Ia).

It will be understood that the definitions for the variables R^{1a}, R^{2a} and R^{3a} given above for the compounds of formulae (Ia) and (IIa) also apply with respect other compounds containing these groups, which are disclosed in the following as starting compounds or intermediates in preferred synthetic procedures of the invention. Furthermore, it will be evident that the groups R^{1a}, R^{2a} and R^{3a} correspond to preferred subgroups of the definitions given for R¹, R² and R³ above. Thus, particularly preferred embodiments of R¹, R² and R³ disclosed herein which are encompassed at the same time by R^{1a}, R^{2a} and R^{3a} will of course be particularly preferred also as embodiments of R^{1a}, R^{2a} and R^{3a}.

Thus, exemplary preferred compounds of formula (I) which can be conveniently prepared in accordance with the process of the invention are:
- *N*-(4-chloro-1-(2-hydroxyethyl)-1*H*-indol-3-yl)-2-cycloheptylacetamide,
- *N*-(4-bromo-1-(2-hydroxyethyl)-1*H*-indol-3-yl)-2-cycloheptylacetamide,
- *N*-(4-chloro-1-(2-hydroxyethyl)-1*H*-indol-3-yl)-2-cyclohexylacetamide,
- *N*-(4-bromo-1 -(2-hydroxyethyl)-1*H*-indol-3-yl)-2-cyclohexylacetamide,
- *N*-(4-chloro-1-(2-hydroxypropyl)-1*H*-indol-3-yl)-2-cycloheptylacetamide,
- *N*-(4-bromo-1 -(2-hydroxypropyl)-1*H*-indol-3-yl)-2-cycloheptylacetamide,
- N-(4-ch!oro-1-(2-hydroxypropyl)-1H-indol-3-yl)-2-cyclohexylacetamide,
- N-(4-bromo-1-(2-hydroxypropyl)-1 H-indol-3-yl)-2-cyclohexylacetamide,
- 2-(bicyclo[2.2.2]octan-1-yl)-N-(4-chloro-1-(2-hydroxypropyl)-1H-indol-3-yl)acetamide,
- 2-(bicyclo[2.2.2]octan-1-yl)-N-(4-bromo-1-(2-hydroxypropyl)-1 H-indol-3-yl)acetamide,
- N-(4-chloro-1-(2-hydroxypropyl)-1H-indol-3-yl)-3-cyclohexylpropanamide,
- N-(4-bromo-1 -(2-hydroxypropyl)-1H-indol-3-yl)-3-cyclohexylpropanamide,
- N-(4-chloro-1 -(2-hydroxypropyl)-1H-indol-3-yl)-3-cycloheptylpropanamide,
- N-(4-bromo-1-(2-hydroxypropyl)-1H-indol-3-yl)-3-cycloheptylpropanamide,
- N-(4-chloro-1-(1,3-dihydroxypropan-2-yl)-1H-indol-3-yl)-2-cyclohexylacetamide,
- N-(4-bromo-1-(1,3-dihydroxypropan-2-yl)-1H-indol-3-yl)-2-cyclohexylacetamide,
- N-(4-chloro-1-(1,3-dihydroxypropan-2-yl)-1H-indol-3-yl)-2-cycloheptylacetamide,
- N-(4-bromo-1-(1,3-dihydroxypropan-2-yl)-1H-indol-3-yl)-2-cycloheptylacetamide,
- 2-(bicyclo[2.2.2]octan-1-yl)-N-(4-chloro-1-(1,3-dihydroxypropan-2-yl)-1 H-indol-3-yl)acetamide
- 2-(bicyclo[2.2.2]octan-1-yl)-N-(4-bromo-1-(1,3-dihydroxypropan-2-yl)-1H-indol-3-yl)acetamide,
- N-(4-chloro-1-(1,3-dihydroxypropan-2-yl)-1 H-indol-3-yl)-3-cyclohexylpropanamide,
- N-(4-bromo-1-(1,3-dihydroxypropan-2-yl)-1H-indol-3-yl)-3-cyclohexylpropanamide,
- N-(4-chloro-1-(1,3-dihydroxypropan-2-yl)-1 H-indol-3-yl)-3-cycloheptylpropanamide, and
- N-(4-bromo-1-(1,3-dihydroxypropan-2-yl)-1H-indol-3-yl)-3-cycloheptylpropanamide.

The rearrangement reaction of the compound of formula (II) or of the preferred compound of formula (IIa), which yields the compound of formula (I) or (Ia), respectively, is generally induced by an acid. Organic or inorganic acids can be used, but organic carboxylic acids are generally preferred. Acetic acid or trifluoroacetic acid (TFA) can be cited as examples of useful acids. These acids can be added to the compound of formula (II) e.g. in neat form.

The reaction generally proceeds under heating, for example at temperatures from 40 °C up to the reflux temperature of the solvent. Reaction times can be suitably adjusted by the skilled person, they typically range from 10 min to 10 h, preferably 30 min to 5 h. The product of formula (I) or (Ia) can be recovered from the reaction mixture, dried and purified by conventional means, such as extraction using organic solvents, silica gel chromatography, etc.

The compounds of formula (II) or (IIa) can be conveniently prepared via reaction of a ketone with hydroxylamine to yield the oxime (ketoxime) of formula (II) or (IIa). It is possible to introduce or suitably convert one or more of the groups R¹ to R⁶ or R^{1a} to R^{3a} after the reaction of the ketone with hydroxylamine. However, it is preferred that the ketone as the starting compound has the formula (III), and more preferably the formula (IIIa): 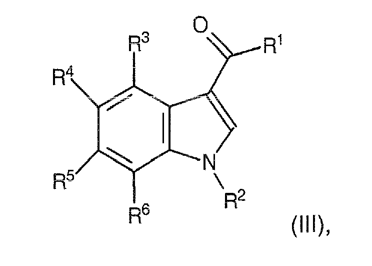 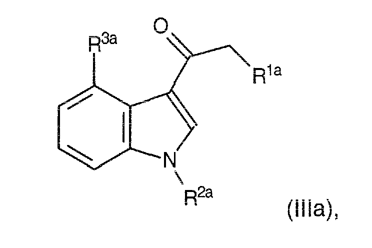 wherein the variables R¹, R^{1a}, R², R^{2a}, R³, R^{3a} as well as R⁴, R⁵ and R⁶ are defined as above, including all preferred definitions and combinations of these.

The reaction of the ketone with hydroxylamine is generally carried out in an organic solvent, such as an alcohol or an ether solvent. It proceedes typically under heating, e.g. at temperatures ranging from 40 °C to the boiling point of the solvent. Reactions times can be suitably adjusted, they typically range from 10 min to 10 h. To ensure optimum yields, it is preferable to use a molar excess of the hydroxylamine. It will be understood that reference to hydroxylamine as a reactant in this context includes the acid addition salts of hydroxyiamine, such as hydroxylamine.HCl. In the latter case, a base such as pyridine can be suitably used to set the hydroxylamine free. The oxime resulting from the reaction can be can be recovered from the reaction mixture, dried and purified by conventional means, such as extraction using organic solvents, trituration of the extracted product with hydrocarbons, etc.

The substituent R² or R^{2a} in the compounds of formulae (II) or (IIa) can be bound to the nitrogen atom of the indole ring system at any convenient stage of their preparation. However, it has been found to be convenient to proceed by providing a compound of formula (IV), preferably formula (IVa) : 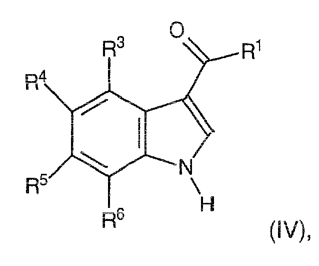 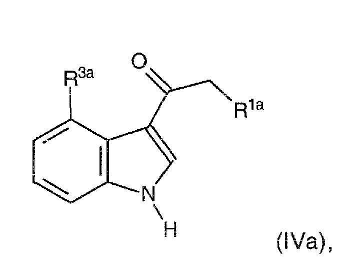 wherein the variables R¹, R^{1a}, R³, R^{3a} as well as R⁴, R⁵ and R⁶ are defined as above, including all, preferred definitions and combinations of these, and subjecting it to a reaction which replaces the hydrogen atom on the ring N-atom with a group R² to yield a compound of formula (III) or (IIIa), respectively.

The reaction which replaces the hydrogen atom on the ring N-atom with a group R² can use a variety of suitable reactants known in the art to form a covalent bond between a nitrogen atom and an alkyl group or a substituted alkyl group. Examples to be mentioned are the reaction of the cyclic amine with an epoxy compound, which yields a hydroxyalkyl group as R², or the reaction of the amine with an alkylhalogenide R²X (with X being, for example I, Br or Cl) which may carry further substituents in the group R² in accordance with the above. A particularly effective way of introducing an alkyl group terminally substituted with a hydroxy group as R² is the reaction of the amine, in particular of the amine of formula (IV) or (IVa) above, with a cyclic alkylene carbonate. Thus, the process according to the invention preferably encompasses the step of converting a starting compound wherein the N-heteroatom in the indole ring system carries a hydrogen atom to a compound wherein the N-heteroatom in the indole ring system carries as substituent R² an alkyl group terminally substituted with an -OH group via reaction of the starting compound with an alkylene carbonate. Examples of suitable alkylene carbonates are ethylene and propylene carbonate, and ethylene carbonate is preferably used to yield a group -CH₂-CH₂OH as R². The reaction proceeds at a high yields especially if a molar excess of the alkylene carbonate over the amine is used. Furthermore, it is preferred to react the amine and the alkylene carbonate in the presence of a non-nucleophilic base, such as diazabicycloundecene (DBU). The reaction can be carried out under heat, typically above the boiling point of the alkylene carbonate, e.g. at temperatures between 50 and 150 °C. Reaction times can be suitably adjusted, e.g. from 1h to 10h.

If the preparation of the compound of formula (I) or (Ia) above starts from an indole compound which is not functionalized at position 3 of the indole heterocycle, a ketone group can be bound to the heterocycle in this position using an acylation reaction as it is known in the art. In the context of the process in accordance with the invention, the acylation is preferably carried out using a compound of formula (V) or (Va) as a starting compound: 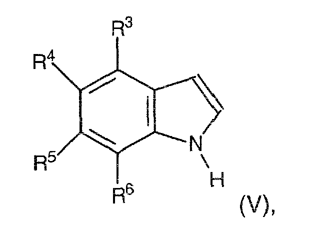 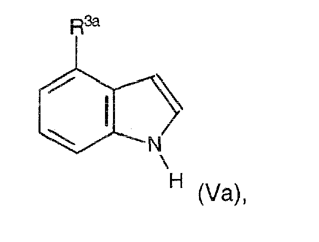 wherein the variables R³, R^{3a}, R⁴, R⁵ and R⁶ are defined as above, including all preferred definitions and combinations of these, to yield a compound of formula (IV) or (IVa).

As acylating reactant, an organic acid of the formula R¹-C(O)-OH or an activated derivative thereof which contains the acyl group -C(O)-R¹, wherein R¹ is a hydrocarbyl group, is generally used. Suitable activated derivatives are those known in the art for acylation reactions. They include acyl halides of the formula R¹-C(O)-X, with X being preferably Cl, and acid anhydrides containing the group -C(O)-R¹. As anhydrides, those of the formula R¹-O-C(O)-O-R¹ are preferred, but mixed anhydrides with only one group R¹ can in principle be used as well. Generally, acyl halides are preferred as acylating reactants. The above applies accordingly in cases where R¹ has the preferred structure -CH₂-R^{1a}.

The acylation reaction is generally carried out in the presence of a catalyst which is adapted to the acylating reactant using principles of synthesis known in the art (cf. March's Advanced Organic Chemistry, 6th edition, 2007, pp. 719). For example, Lewis acid catalysts are commonly used if the acylation reactant is an acyl halide. Examples of suitable catalysts are AlCl₃ or SnCl₄. The acylation reaction is generally carried out in a sutiable solvent, such as dry benzene, and preferably at or below room temperature, e.g. 0°C. The resulting ketone can be can be recovered from the reaction mixture, dried and purified by conventional means, such as extraction using organic solvents, trituration of the extracted product with hydrocarbons, etc.

From the above, it will be understood that a preferred process for the preparation of a compound of formula (I) in accordance with the invention comprises the steps of
i) providing a compound of formula (V): 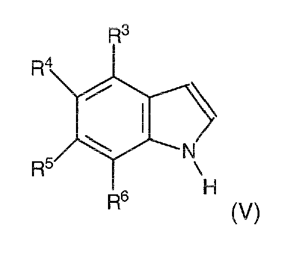 wherein R³, R⁴, R⁵ and R⁶ are independently selected from H, alkyl, alkoxy, CN or CF₃ and halogen,
ii) acylating the compound of formula (V) with an organic acid of the formula R¹-C(O)-OH or an activated derivative thereof which contains the acyl group -C(O)-R¹, wherein R¹ is a hydrocarbyl group, to yield a compound of formula (IV) 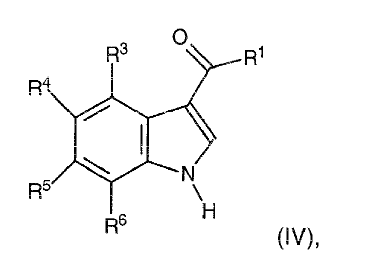 wherein R¹, R³, R⁴, R⁵, R⁶ are defined as above,
iii) subjecting the compound of formula (IV) to a reaction which replaces the hydrogen substituent on the ring N-atom with a group R², to yield a compound of formula (III), 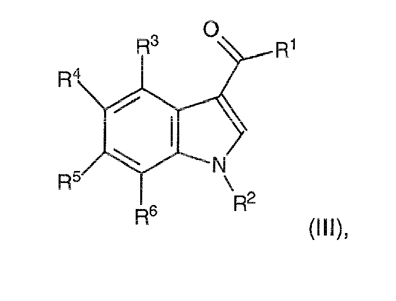 wherein R¹, R³, R⁴, R⁵ and R⁶ are defined as above, and R² is an alkyl group which is optionally substituted by hydroxy, alkoxy, halogen, -NR⁷R⁸, CN or CF₃, with R⁷and R⁸ being independently selected from H or alkyl,
iv) reacting the compound of formula (III) with hydroxylamine to yield a compound of formula (II) 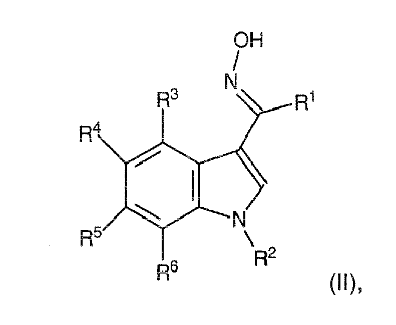 wherein R¹, R², R³, R⁴, R⁵ and R⁶ are defined as above, and
v) allowing the compound of formula (II) to undergo a rearrangement reaction to yield the compound of formula (I).

It will be understood that the preferred meanings defined above for R¹ to R⁶ also apply for this preferred reaction scheme.

Further preferred is a process for the preparation of a compound of formula (la) in accordance with the invention, which comprises the steps of
i) providing a compound of formula (Va): 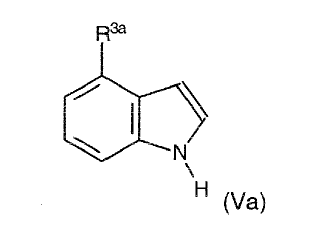 wherein R^{3a} is selected from alkyl and halogen,
ii) acylating the compound of formula (Va) with an organic acid of the formula R^{1a}-CH₂-C(O)-OH or an activated derivative thereof which contains the acyl group -C(O)-CH₂-R^{1a}, wherein R^{1a} is selected from cyclopentyl, cyclopentylmethyl, cyclohexyl, cyclohexylmethyl, cycloheptyl, cycloheptylmethyl, bicyclo[2.2.2]octan-1-yl and bicyclo[2.2.2]octan-1-ylmethyl, to yield a compound of formula (IVa) 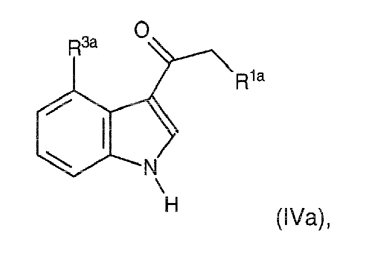 wherein R^{1a} and R^{3a} are defined as above,
iii) subjecting the compound of formula (IVa) to a reaction which replaces the hydrogen substituent on the ring N-atom with a group R^{2a}. to yield a compound of formula (IIIa), 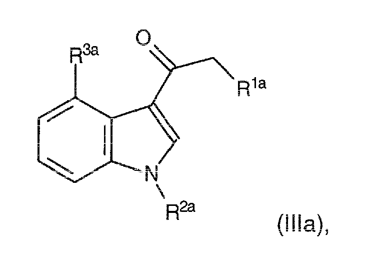 wherein R^{1a} and R^{3a} are defined as above, and R^{2a} is an alkyl group which is optionally substituted by hydroxy,
iv) reacting the compound of formula (IIIa) with hydroxylamine to yield a compound of formula (IIa) 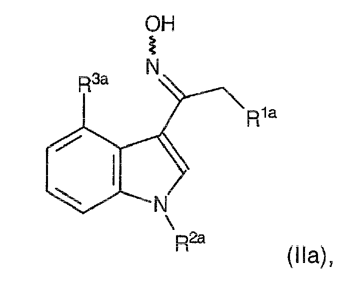 wherein R^{1a}, R^{2a} and R^{3a} are defined as above, and
v) allowing the compound of formula (IIa) to undergo a rearrangement reaction to yield the compound of formula (Ia).

It will be understood that the preferred meanings defined above for R^{1a} to R^{3a} also apply for this preferred reaction scheme.

Pharmaceutically acceptable salts of the compounds prepared in accordance with the invention can be formed, e.g., via protonation of the N-atom of the indole ring system or of other optional amine substituents. They include salts formed with organic and inorganic anions such as those derived from hydrochloric, phosphoric, acetic, oxalic, tartaric acid, etc.

The following examples are intended to illustrate the invention, but should not be construed to impose a imitation on the broader description provided above or on the annexed claims.

### EXAMPLES

### Example 1.

Target Compound:

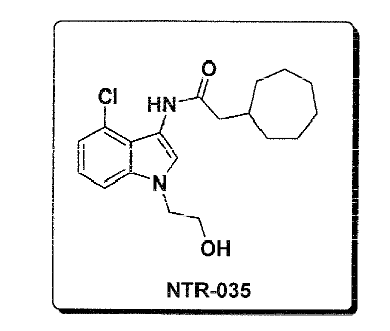

Synthetic scheme:

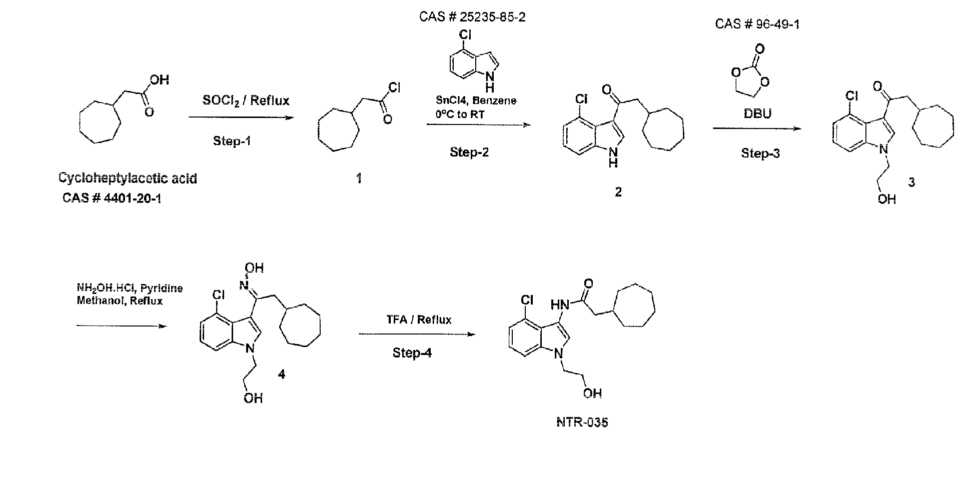

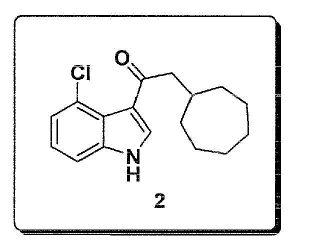

**Preparation of 1-(4-chloro-1H-indol-3-yl)-2-cycloheptylethanone (Compound-2):** Part-A: Synthesis of Cycloheptylacetylchloride (Compound-1): Cycloheptylacetic acid (75g, 0.48 mol, Alfa aesar, Lot # 10124299) was placed in a 1 L single neck round bottom flask fitted with a reflux condenser. To this SOCl₂ (122mL, 1.69 mol) was added at rt and then refluxed for 1.5h. The excess SOCl₂ was removed under atmospheric pressure at 80°C bath temperature until distillation stops, then applied vacuum (∼12mm) at 80° C bath temperature for 1 h. The crude acid chloride was dissolved in dry benzene (150mL) and used for the Friedel-Crafts reaction.

Part-B: Preparation of Compound-2: To a stirred solution of 4-chloroindole (50g, 0.33 mol, AVIAN International Limited, Lot # 20090713) in dry benzene (1200mL) [placed in a 3 neck round bottom flask fitted with mechanical stirrer fitted with an addition funnel] was added cycloheptylacetyl chloride dissolved in dry benzene (Compound-1, prepared in Part-A) at 0 °C over a period of 30min. The reaction mixture stirred for 10min at 0 °C. To this mixture stannic chloride (172g, 0.66 Mol, Spectrochem, Lot # 3253294) dissolved in dry Benzene (150mL) was added slowly over a period of 1h at 0 °C. The mixture was stirred for 1 h at rt. TLC showed completion of the reaction (TLC sample preparation: A small sample was drawn from the reaction mixture and quenched with water, basified with saturated NaHCO₃ and then extracted with ethyl acetate, mobile phase: 35% EtOAc / Hexane, Rf = 0.2). 1 N HCl (500mL) was added to the reaction mixture slowly over a period of 10min at rt and the mixture was extracted with ethyl acetate (3 x 500mL). The first extraction yielded an emulsion which was separated, stirred with 10% methanol /ethyl acetate (300mL) over night to obtain a clear layer. The combined organic extracts were washed with water (3 x 800mL), sat. sodium bicarbonate solution (500mL), brine (300mL), dried over anhydrous sodium sulfate and concentrated to obtain the crude product which was triturated with diethyl ether (100mL) for 15min and filtered. The solid was washed with diethyl ether (50mL) and dried under vacuum for 1h (white crystalline solid, 50g, 52.5%). ¹HNMR (DMSO-D6): δ 12.1 (broad s, 1H), 8.3 (s, 1H), 7.4 (dd, 1H), 7.1-7.2 (m, 2H), 2.7-2.8 (d, 2H), 2.0-2.1 (m, 1 H), 1.1-1.8 (m, 12H), HLC purity ∼99.53 % and melting range 165-168°C.

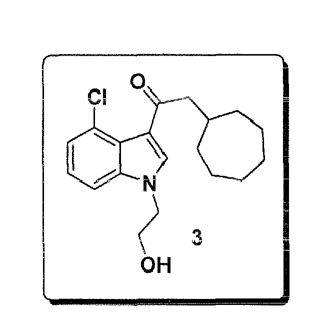

**Preparation of 1-(4-chloro-1-(2-hydroxythy)-1H-indol-3-yl)-2-cycloheptylethanone (Compound-3):** Compound-2 (50g, 0.173mol) and ethylene carbonate (45.6g, 0.519mol, Aldrich, Lot # S80704-499) were placed in a single neck 1L round bottom flask. DBU (26.2g, 0.173 mol, Chemlabs, Lot # 5244687) was added and the mixture was slowly heated to 95°C over a period of 30min and was stirred at 95 °C for 8h. TLC showed completion of reaction (TLC sample preparation: A small sample of the reaction mixture was diluted with water and extracted with ethyl acetate, mobile phase: 40% Ethyl acetate/hexane, Rf= 0.4). The reaction mixture was cooled to rt and water (150mL) was added and stirred for 16h. The solid product was filtered and dried under vacuum for 3h (white powder, 54g, 93.7%). ¹HNMR (DMSO-D6): δ 8.3 (s, 1 H), 7.5-7.6 (dd, 1H), 7.2 (m, 2H), 4.9 (t, 1H), 4.2-4.3 (t, 2H), 3.7-3.8 (m, 2H), 2.7-2.8 (d, 2H), 2.0-2.1 (m, 1 H), 1.2-1.75 (m, 12H). HPLC purity ∼95.87% and melting range: 113-119 °C.

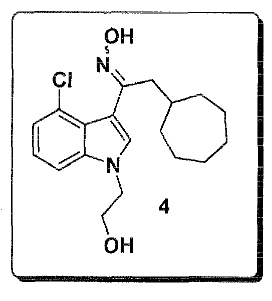

**Preparation of 1-(4-chloro-1-(2-hydroxyethyl)-1H-indol-3-yl)-2-cycloheptylethanone oxime (Comoound-4):** To a solution of compound-3 (54g, 0.162 mol) in methanol (270mL) were added hydroxylamine. HCl (55.9g, 0.810mol, Rankem, Lot # J019L08) and pyridine (38.4g, 0.486mol, Merck, Lot # SE9S590204) at rt and stirred for 10min. The mixture was heated at reflux for 4h. TLC showed completion of the reaction (TLC preparation: Reaction mixture diluted with Ethyl acetate, mobile phase: 45% Ethylacetate / Hexane, Rf: 0.4 (major isomer) & 0.5 (minor isomer)). The reaction mixture was concentrated and the residue was diluted with water and extracted with ethyl acetate (3 x 300mL). The combined organic layers were washed with water (500mL), brine (300mL), dried over sodium sulfate and concentrated to obtain the crude product that was triturated with hexane (100mL) to obtain the desired product as off white solid (55g, 97%, mixture of E/Z isomers). HPLC purity ∼90.7% and melting point: 168-173 °C.

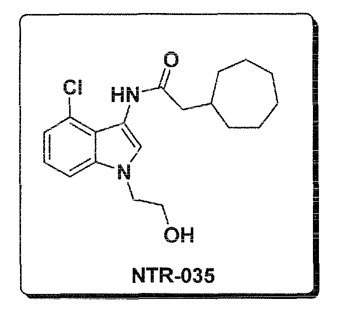

**Preparation of N-(4-chloro-1-(2-hydroxyethyl)-1H-indol-3-yl)-2-cycloheptylacetamide (NTR-035):** Compound-4 (55g, 0.158mol) was placed in 1 L single neck round bottom flask and trifluoroacetic acid (275mL, Merck, Lot # AG7A570451) was added at rt. The mixture was heated to reflux over a period of 30min and maintained at reflux for 1h. TLC showed completion of the reaction (TLC sample preparation: reaction mixture diluted with water, basified with NaHCO₃ and then extracted with ethyl acetate. Mobile phase: 35% Ethyl acetate/Chloroform, product is at 0.4 Rf and trifluoroacetamide is at 0.8 Rf). The reaction mixture was concentrated, diluted with water and treated with saturated sodium bicarbonate solution to pH= 7-8) and extracted with chloroform (3 x 300mL). The combined organic layers were washed with water (500mL), brine (300mL), dried over anhydrous sodium sulfate and concentrated to obtain the crude product (65g, mixture NTR-035 and trifluoroacetamide derivative). After the addition of methanol (200mL) and K₂CO₃ (65g) the mixture was stirred at rt for 30min. TLC showed conversion of the corresponding trifluoroacetamide to NTR-035 (TLC: reaction mixture directly checked). The reaction mixture was diluted with water (400mL) and extracted with chloroform (2 x 250mL). The combined organic layers were washed with water (2 x 100mL), brine (1 x 100mL), dried over anhydrous sodium sulfate and concentrated to yield the crude product as a solid (55g). Purification over silica gel (120g, 100-200mesh) by column chromatography (packed with 800g of 100-200mesh silica gel) using 15% Ethyl acetate/Chloroform yielded NTR 35 (29g 53%). ¹HNMR (DMSO-D6): δ 9.0-9.1 (s, 1H), 7.5-7.6 (s, 1H), 7.4-7.5 (d, 1H), 7.0-7.2 (t, 1 H), 6.9-7.0 (d, 1H), 4.8-4.9 (t, 1H), 4.1-4.2 (t, 2H), 3.6-3.7 (m, 2H), 2.2-2.3 (d, 2H), 2.0 (m, 1H), 1.1-1.8 (m, 12H). HPLC purity: 99.5%, melting range: 108-110°C

Example 2:

### Target Compound:

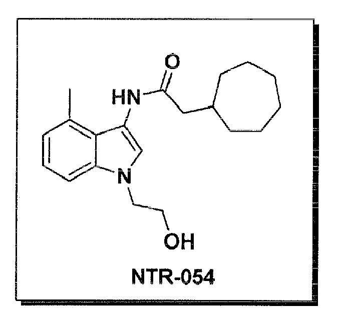

Synthetic scheme:

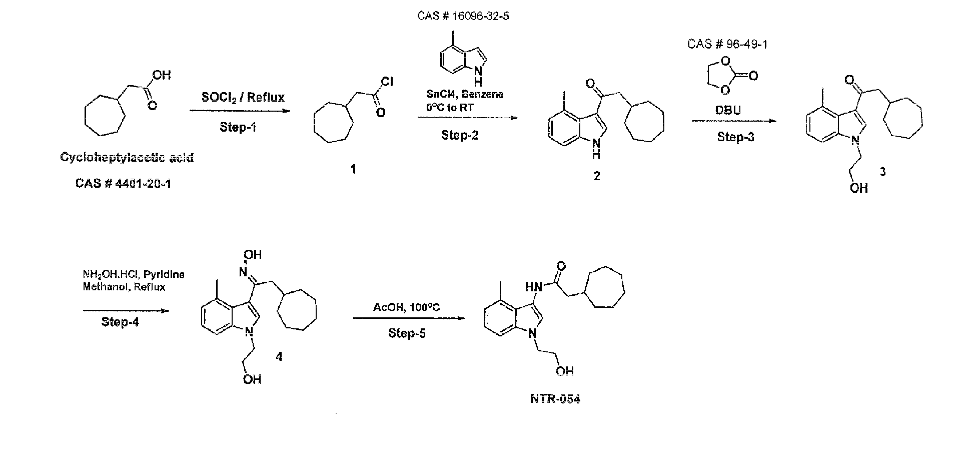

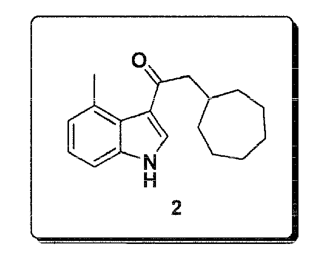

**Preparation of 2-cycloheptyl-1-(4-methyl-1H-indol-3-yl)ethanone (Compound-2):** Part-A: Synthesis of 2-cycloheptylacetyl chloride (Compound-1): Thionyl Chloride (5mL, 0.067mol) was added to cycloheptylacetic acid (2.25g, 0.0144 mol, Alfa aesar, Lot # 10124299) at rt and heated to reflux for 1.5h. Excess SOCl₂ was removed under atmosphere pressure at 80°C bath temperature and dried under vacuum (∼12mm) for 1h. The crude acid chloride was dissolved in dry benzene (10mL) and used for Friedel-Crafts reaction.

Part-B: Preparation of Compound-2: To a stirred solution of 4-methylindole (1.5g, 0.0114 mol, Molekula Life Sciences Ltd, Lot # M10431/04-09) in dry benzene (10mL) was added cycloheptylacetyl chloride dissolved in dry benzene (Compound-1, prepared in Part-A) at 0 °C over a period of 5min and stirred for 10min at 0 °C. A solution of stannic chloride (5.9g, 0.0228 mol, Spectrochem, Lot # 3253294) in dry benzene (10mL) was added slowly over a period of 10min at 0 °C. The mixture was allowed to stir for 1 h at rt. 1 N HCl (10mL) was added slowly and the mixture was stirred for 10min followed by extraction with ethyl acetate (3 x 50mL). The combined organic layers were washed with water (2 x 20mL), sat.sodium bicarbonate solution (20mL), brine (20mL), dried over anhydrous sodium sulfate and concentrated. The crude product was triturated with 20% diethyl ether in hexane to obtain the pure compound as a brown solid (1.4g, 45%). ¹HNMR (CDCl₃): δ 8.5 (br, 1H), 7.8 (s, 1H), 7.15-7.25 (m, 2H), 7.0-7.1 (d, 1H), 2.8-2.9 (s, 3H), 2.7-2.8 (d, 2H), 2.3-2.4 (m, 1H), 1.7-1.8 (m, 2H), 1.4-1.7 (m, 8H), 1.2-1.35 (m, 2H). HPLC purity ∼99.45% and Melting range is 146-150°C

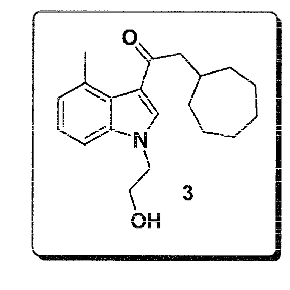

**Preparation of 2-cycloheptyl-1-(1-(2-hydroxyethyl)-4-methyl-1H-indol-3-yl)ethanone (Compound-3):** A mixture of Compound-2 (1.42g, 0.0055mol), ethylene carbonate (2.9g, 0.033mol, Aldrich, Lot # S80704-499) and DBU (0.847g, 0.0055 mol, Chemlabs, Lot # 5244687) was heated for 8h to 95°C. The reaction mixture was cooled to rt, diluted with water (25mL) and stirred for 4h. The solid product was filtered, washed with water and dried under vacuum to obtain the product as off white solid (1.4g, 86%). ¹HNMR (CDCl₃): δ 7.8 (s, 1 H), 7.1-7.2 (m, 2H), 7.0-7.1 (d, 1H), 4.3 (t, 2H), 4.0 (m, 2H), 2.8 (s, 3H), 2.65-2.7 (d, 2H), 2.1-2.25 (m, 1H), 2.0 (m, 1H), 1.7-1.8 (m, 2H), 1.4-1.7 (m, 8H), 1.15-1.3 (m, 2H). HPLC purity ∼98.7% and Melting range: 98-104 °C.

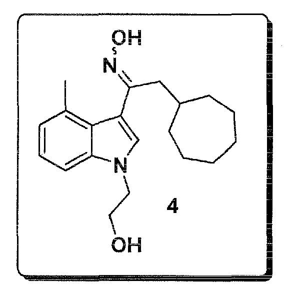

**Preparation of 1-(4-chloro-1-(2-hydroxyethyl)-1H-indol-3-yl)-2-cycloheptylethanone oxime (Compound-4):** To a solution of Compound-3 (1.4g, 0.0045mol) in methanol (30mL) were added hydroxylamine.HCl 2.4g, 0.0356mol, Rankem, Lot # J019L08) and pyridine (1.761g, 0.022mol, Merck, Lot # SE9S590204) at rt. The mixture was heated to reflux for 6hr. The solvent was removed and the residue was diluted with water and extracted with ethyl acetate (3 x 25mL). The combined organic layers were washed with water (25mL), brine (25mL), dried over sodium sulfate and concentrated to obtain the crude product. This crude material was triturated with hexane (5mL) to obtain the desired product as pale yellow solid (1.3g, 87%, mixture of E/Z isomers). HPLC purity ∼90.9% and melting range is 120-123°C.

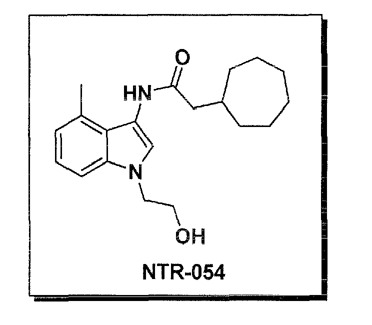

**Preparation of N-(4-chloro-1-(2-hydroxyethyl)-1H-indol-3-yl-2-cycloheptylacetamide (NTR-054):** A solution of compound-4 (1.8g, 0.00392mol) in acetic acid (6mL) was heated to 100°C for 2h. The reaction mixture was diluted with water and extracted with ethyl acetate (2 x 50mL). The combined organic layers were washed with water (2 x 20mL), brine (1 x 20mL), dried over anhydrous sodium sulfate and concentrated. The residue was dissolved in methanol (15mL) K₂CO₃ (1.5g, 0.011 mol) was added and the mixture was stirred for 30min at rt followed by the addition of water (15mL) and extraction with chloroform (3x15mL). The combined organic layers were washed water (15mL), brine (10mL), dried over sodium sulfate and concentrated to obtain the crude product. Purification over silica gel (100-200mesh) with ethyl acetate and hexane yielded the product (520mg, 40%). ¹HNMR (DMSO-D6): δ 9.0-9.1 (s, 1H), 7.2-7.3 (m, 2H), 6.9-7.0 (t, 1H), 6.78 (d, 1H), 4.8-4.9 (t, 1H), 4.1-4.2 (t, 2H), 3.6-3.7 (m, 12H), 2.5-2.6 (s, 3H), 2.2 (d, 2H), 2.0 (m, 1H), 1.2-1.8 (m, 12H). melting range is 79-83°C

## Claims

1. A process for the preparation of a compound of formula (I) 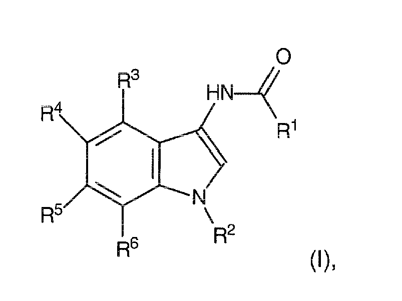 wherein R¹ is a hydrocarbyl group, R³, R⁴, R⁵ and R⁶ are independently selected from H, alkyl, alkoxy, CN or CF³ and halogen, and R² is an alkyl group which is optionally substituted by hydroxy, alkoxy, halogen, -NR⁷R⁸, CN or CF₃, with R⁷ and R⁸ being independently selected from H or alkyl,
comprising the rearrangement reaction of a compound of formula (II) 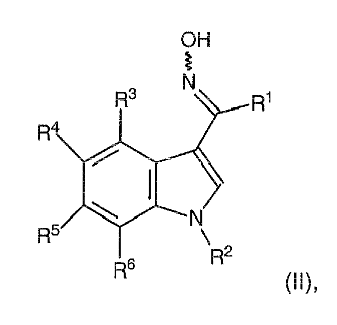 wherein R¹, R², R³, R⁴, R⁵ and R⁶ are defined as above.

2. The process of claim 1, wherein the compound of formula (II) is prepared by a process comprising a step of reacting a compound of formula (III) 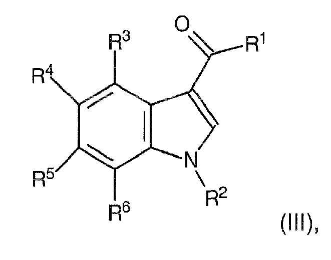 with hydroxylamine,
wherein, in formula (III), R¹ is a hydrocarbyl group, R³, R⁴, R⁵ and R⁶ are independently selected from H, alkyl, alkoxy, CN or CF₃ and halogen, and R² is an alkyl group which is optionally substituted by hydroxy, alkoxy, halogen, -NR⁷R⁸, CN or CF₃, with R⁷ and R⁸ being independently selected from H or alkyl.

3. The process of claim 2, wherein the compound of formula (III) is prepared by a process comprising a step of subjecting a compound of formula (IV) 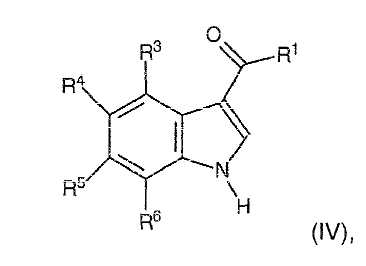 wherein R¹ and R³ to R⁶ are defined as above, to a reaction which replaces the hydrogen substituent on the ring N-atom with a group R², to yield a compound of formula (III), 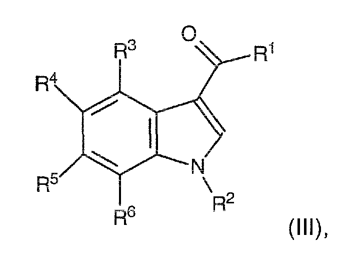 wherein R¹ is a hydrocarbyl group, R³, R⁴, R⁵ and R⁶ are independently selected from H, alkyl, alkoxy, CN or CF₃ and halogen, and R² is an alkyl group which is optionally substituted by hydroxy, alkoxy, halogen, -NR⁷R⁸, CN or CF₃, with R⁷ and R⁸ being independently selected from H or alkyl.

4. The process of any of claims 1 to 3 above, further comprising the acylation of a compound of formula (V) 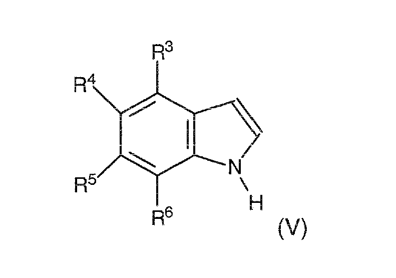 wherein R³ R⁴, R⁵ and R⁶ are independently selected from H, alkyl, alkoxy, CN or CF₃ and halogen, with an organic acid of the formula R¹-C(O)-OH or an activated derivative thereof which contains the acyl group -C(O)-R¹, wherein R¹ is a hydrocarbyl group.

5. The process of claim 4, wherein the acylation is carried out with an acid halide of the formula R¹-C(O)-X in the presence of a Lewis acid catalyst.

6. The process of claim 5, wherein the Lewis acid catalyst is SnCI_{4.}

7. The process of any of claims 1 to 6, wherein the compound of formula (I) is a compound of formula (Ia) 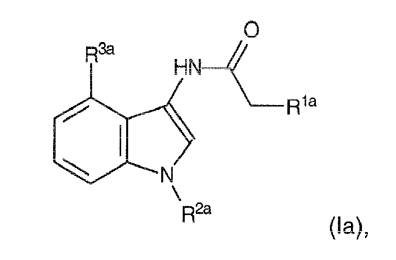 wherein R^{3a} is alkyl or halogen; R^{1a} is selected from cyclopentyl, cyclopentylmethyl, cyclohexyl, cyclohexylmethyl, cycloheptyl, cycloheptylmethyl, bicyclo[2.2.2]octan-1-yland bicyclo[2.2.2]octan-1-ylmethyl; and R^{2a} is an alkyl group which is optionally substituted by hydroxy; and is prepared by a process comprising the rearrangement of a compound of formula (IIa): 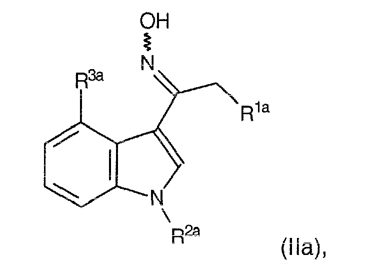 wherein R^{1a}, R^{2a} and R^{3a} are defined as for the compound of formula (Ia).

8. The process of any of claims 1 to 6, comprising the step of converting a starting compound wherein the N-heteroatom in the indole ring system carries a hydrogen atom to a compound wherein the N-heteroatom in the insole ring system carries a group -CH₂-CH₂-OH as a substituent via reaction of the starting compound with ethylene carbonate.

9. The process of claim 8, wherein the reaction with ethylene carbonate is carried out in the presence of a non-nucleophilic base.

10. The process of claim 8 or 9, wherein the starting compound which is reacted with ethylene carbonate is a compound of formula (IV).

11. The process of claim 1, comprising the steps of
i) providing a compound of formula (V): 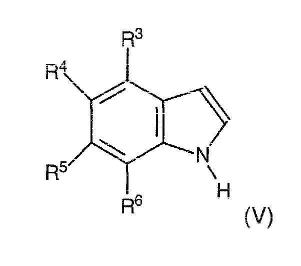 wherein R³, R⁴, R⁵ and R⁶ are independently selected from H, alkyl, alkoxy, CN or CF₃ and halogen,
ii) acylating the compound of formula (V) with an organic acid of the formula R¹-C(O)-OH or an activated derivative thereof which contains the acyl group -C(O)-R¹, wherein R¹ is a hydrocarbyl group, to yield a compound of formula (IV) 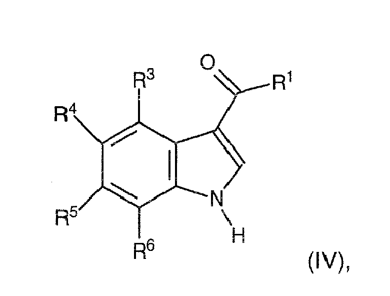 wherein R¹, R³, R⁴, R⁵, R⁶ are defined as above,
iii) subjecting the compound of formula (IV) to a reaction which replaces the hydrogen
substituent on the ring N-atom with a group R², to yield a compound of formula (III), 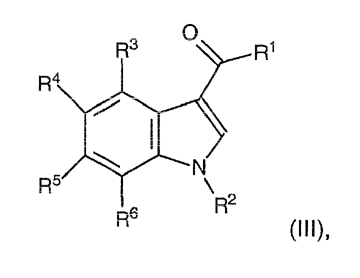 wherein R¹, R³, R⁴, R⁵ and R⁶ are defined as above, and R² is an alkyl group which is optionally substituted by hydroxy, alkoxy, halogen, -NR⁷R⁸, CN or CF₃, with R⁷ and R⁸ being independently selected from H or alkyl,
iv) reacting the compound of formula (III) with hydroxyamine to yield a compound of formula (II) 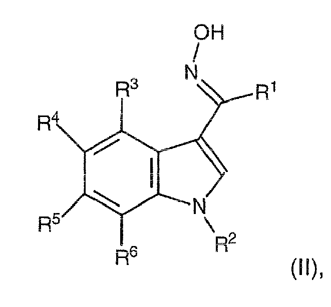 wherein R¹, R², R³, R⁴, R⁵ and R⁶ are defined as above, and v) allowing the compound of formula (II) to undergo a rearrangement reaction to yield the compound of formula (I).

12. The process of claim 11, comprising the step of reacting the compound of formula (IV) with ethylene carbonate to yield a compound of formula (III) wherein R² is -CH₂-CH₂-OH.

13. The process of claim 9 or 10, wherein R³ to R⁶ are independently selected from hydrogen, halogen or methyl and R¹ is a group of formula ―CH₂-R^{1a}, wherein R^{1a} is selected from cyclopentyl, cyclopentylmethyl, cyclohexyl, cyclohexylmethyl, cycloheptyl, cycloheptylmethyl, bicyclo[2.2.2]octan-1-yl and bicyclo[2.2.2]octan-1-ylmethyl.

14. The process of claim 13, wherein R³ is selected from methyl and halogen and R⁴ to R⁶ are hydrogen.

15. A compound of formula (II) 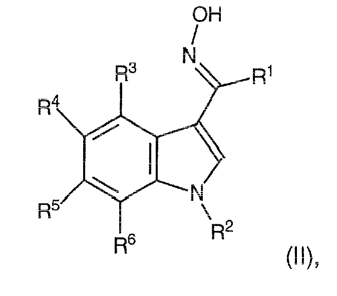 wherein R¹ is a hydrocarbyl group, R³, R⁴, R⁵ and R⁶ are independently selected from H, alkyl, alkoxy, CN or CF₃ and halogen, and R² is an alkyl group which is optionally substituted by hydroxy, alkoxy, halogen, -NR⁷R^{B}, CN or CF₃, with R⁷ and R⁸ being independently selected from H or alkyl.
